# EUROPEAN PATENT APPLICATION

(11) **EP 1 653 204 A1**
(43) Date of publication of application: **03.05.2006**
(21) Application number: 05110161.6
(22) Date of filing: 28.10.2005
(51) Int. Cl.: G01J 1/42

(54) **Device and method for determining a personalized sun exposure program**

(30) Priority: 29.10.2004 IT MI20042081
(71) Applicant: Derming S.r.l., 20052 MONZA (IT)
(72) Inventor: Setaro, Michele, 20052 Monza (IT); Sparavigna, Adele, 20052 Monza (IT); Pasero, Eros Gian Alessandro, 10131 Torino (IT); Riccardi, Marco, 10126 Torino (IT)
(74) Representative: Jorio, Paolo

(57) **Abstract**

A device (1) for determining a personalized sun exposure program has a portable casing (2) housing a processing unit (4) associated with a memory (5); the processing unit (4) is connected to a data-entry keyboard (9), to sun sensors (12), and to a light source (15) associated with a photometric detector (16) for measuring the light emitted by the light source (15) and reflected by a user's skin surface; the processing unit (4) calculates an intensity value (I), representing local sun conditions, on the basis of the measurement supplied by the sensors (12), and calculates a reflection value (R_{D}), representing the user's phototype and skin condition, on the basis of the measurement supplied by the detector (16); the processing unit then supplies a personalized sun exposure program as a function of the reflection value (R_{D}) and the intensity value (I); and the program indicates one or more exposure periods and, for each period, the time of day, the exposure time, and the sun protection factor to be used in that period.

## Description

The present invention relates to a device and method for determining a personalized sun exposure program.

As is known, prolonged exposure to strong sun may produce varying degrees of harm to the skin, to safeguard against which, protective creams or lotions are available, normally containing substances for blocking out or filtering rays in the more harmful regions of the sun's electromagnetic spectrum. Protective creams are available which provide for varying degrees of protection, indicated by a sun protection factor (SPF) according to a general, all-purpose scale.

The problem therefore arises, especially in the case of exposure to the sun for tanning purposes, of making the best use of tanning time to achieve the deepest possible tan without harming the skin. In addition to selecting the degree of protection best suited to one's own skin type ("skin phototype") and actual sun conditions, exposure time and duration must also be assessed. That is, too-high a sun protection factor and/or too-short exposure times result in a poor tan, whereas, conversely, too-low a protection factor and/or excessive exposure or at the wrong time may result in irritating or harmful sunburn, and even skin problems which may eventually degenerate into pigmentation changes, skin changes resulting in premature ageing, precancerous lesions, and skin cancer.

Unaided, people in general are unable to determine the correct sun protection factor to adopt in given sun conditions, or to establish the best sun exposure program. Nor are instruments available to provide this information in a straightforward, fast, low-cost, safe, reliable manner.

It is an object of the present invention to provide a solution to the above problems. More specifically, it is an object of the invention to provide a device and method enabling anyone to determine, easily, cheaply, quickly and reliably, a personalized sun exposure program which, besides indicating which protective product to apply, as a function of local environment conditions and the user's skin type, also indicates the best exposure time to achieve, in the time available to the user, the deepest possible tan with no risk to the skin.

The present invention therefore relates to a device and method of determining a personalized sun exposure program, as claimed in the accompanying Claims 1 and 8 respectively.

The device and method according to the invention provide for determining quickly and easily, but also objectively and reliably, not only the sun protection factor best suited to the user in actual current sun conditions, but also the time periods in which the user may achieve the deepest possible tan without endangering the skin. In other words, the invention allows anyone to establish a personalized sun exposure program to achieve a deep tan in the utmost safety.

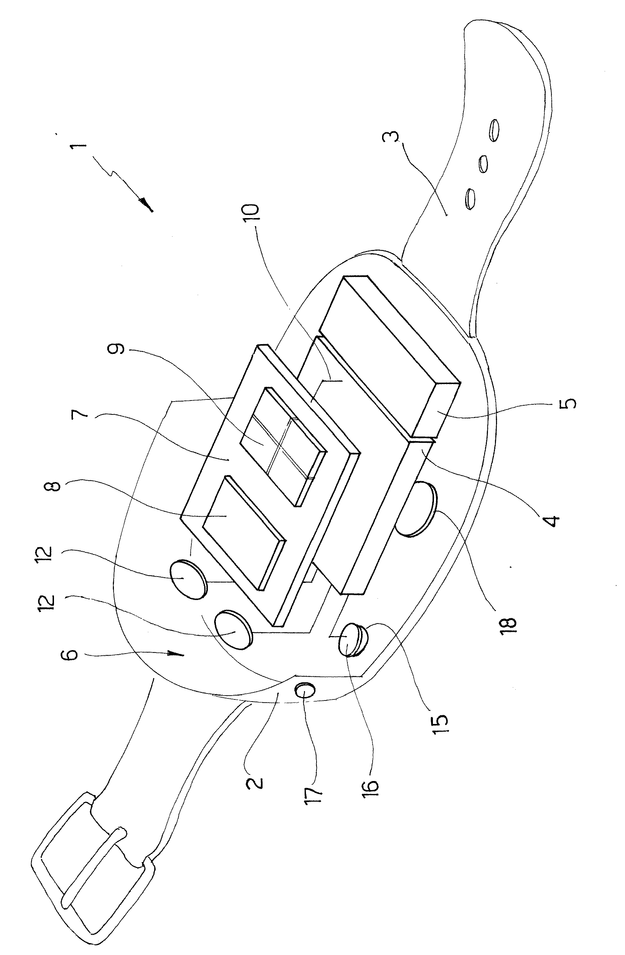

A non-limiting embodiment of the present invention will be described by way of example with reference to the accompanying drawing showing a schematic of a device for determining a personalized sun exposure program in accordance with the invention.

With reference to the accompanying drawing, a device 1 for determining a personalized sun exposure program comprises a portable casing 2 having, in the example shown (but not necessarily), a strap 3 for fastening it to a user's wrist like a bracelet or watch.

Casing 2 houses a processing unit, e.g. a microprocessor, 4 associated with a memory 5. One face, e.g. a top face, 6 of casing 2 has a control panel 7 comprising a screen 8 and a small data-entry keyboard 9 connected to each other and to processing unit 4 by a control circuit 10. Keyboard 9 provides for loading processing unit 4 with various information, in particular the exposure day (i.e. first, second, third, etc.).

Casing 2 is also fitted, e.g. on face 6, with one or more ultraviolet radiation sensors 12 connected to processing unit 4, and which measure the intensity of UV light.

Device 1 also comprises a light source 15 fitted to a face of casing 2 and associated with a photometric detector, e.g. spectrophotometer, 16 for measuring the intensity of the light emitted by light source 15 and reflected by a skin surface. Light source 15 and detector 16 are operated by a switch 17.

Device 1 also comprises an electric power source, e.g. a battery, 18 for powering the various component parts of device 1.

To implement the method of determining a personalized sun exposure program according to the invention, device 1 operates as follows.

The user enters the exposure day D (i.e. the first, second day, etc. of exposure to the sun) on keyboard 9, and turns on light source 15 to illuminate a portion of skin. Detector 16 measures the light emitted by light source 15 and reflected by the illuminated skin portion, and which indicates the user's phototype and instantaneous skin condition : a dark and/or tanned skin reflects less than a light and/or reddened skin. The reflected-light measurement by detector 16 is sent to processing unit 4, which associates it with exposure day D to obtain a reflection value R_{D}.

The user then exposes device 1 to the sun to allow sensors 12 to measure local sun conditions and, in particular, the intensity of UV light.

On the basis of the light intensity value EI measured by sensors 12, processing unit 4 calculates an intensity value I. Processing unit 4 contains instructions for performing this calculation, and advantageously employs a linear proportional function of the light intensity value EI measured by sensors 12 and the intensity value I.

Processing unit 4 calculates the best sun protection factor SPF as a function of value R_{D} (representing the user's phototype and skin condition) and of intensity value I (representing local sun conditions). To calculate the best sun protection factor, processing unit 4 contains memorized instructions, i.e. associates a specific protection factor with each combination of R_{D} and I values, e.g. on the basis of a set table stored in memory 5.

Processing unit 4 then calculates a personalized daily sun exposure program as a function of the user's phototype and instantaneous skin condition, and of the intensity value I indicating local sun conditions. More specifically, the program indicates one or more exposure periods for the day and, for each period, an exposure time interval and/or duration and the sun protection factor to employ in that period.

On the basis of the instructions stored in memory 5, processing unit 4 daily adapts the protection factor to be used and the exposure duration and/or time interval to skin conditions and sun conditions.

That is, on the first exposure day, processing unit 4 supplies a first sun exposure program based on the user's initial skin condition, i.e. phototype, and on local sun conditions. On each successive day, processing unit 4 compares value R_{D} with the value R_{D-1} of the preceding day. The resulting options are as follows:
- if R_{D}<R_{D-1}, this means the user's skin is darker (more tanned) than the day before, so processing unit 4 reduces the protection factor and increases exposure time (alters the duration and/or time interval of the exposure periods);
- if R_{D}=R_{D-1}, this means the user's skin is substantially the same as the day before, so processing unit 4 reduces the protection factor and/or increases exposure time;
if R_{D}>R_{D-1}, this means the user's skin is reddened, so processing unit 4 increases the protection factor and reduces exposure time to prevent sunburn.

The program determined by processing unit 4 is displayed on screen 8.

## Claims

1. A device (1) for determining a personalized sun exposure program, **characterized by** comprising: sun sensor means (12); photometric detecting means (16) for measuring the light reflected by a skin surface; and processing means (4) for:
- calculating an intensity value (I), representing local sun conditions, on the basis of the measurement by said sensor means (12);
- calculating a reflection value (R_{D}), representing the phototype and skin condition of a user, on the basis of the measurement by said detecting means (16);
- calculating an optimum sun protection factor (SPF) as a function of said reflection value (R_{D}) and said intensity value (I).

2. A device as claimed in Claim 1, **characterized by** comprising memory means (5) associated with the processing means (4) and containing instructions for calculating the optimum sun protection factor, i.e. for associating a specific sun protection factor with each combination of reflection (R_{D}) and intensity (I) values.

3. A device as claimed in Claim 2, **characterized in that** the memory means (5) contain instructions for calculating a personalized sun exposure program as a function of said reflection value (R_{D}) and said intensity value (I); said program indicating one or more exposure periods, and, for each period, the time of day, the exposure time, and the sun protection factor to be used **in that** period.

4. A device as claimed in one of the foregoing Claims, **characterized by** comprising a light source (15) associated with a photometric detector (16), such as a spectrophotometer, for measuring the intensity of the light emitted by the light source (15) and reflected by a skin surface.

5. A device as claimed in one of the foregoing Claims, **characterized in that** the processing means (4) periodically adapt the sun protection factor to be used and the exposure duration and/or time interval to skin conditions and sun conditions.

6. A device as claimed in Claim 5, **characterized in that** the processing means (4) contain instructions for comparing said reflection value (R_{D}) with a reflection value (R_{D-1}) of the previous day, and for accordingly adapting the sun exposure program.

7. A device as claimed in one of the foregoing Claims, **characterized by** comprising a portable casing (2) housing the sensor means (12), the detecting means (16), the processing means (4), and electric power supply means (18).

8. A method of determining a personalized sun exposure program, the method being **characterized by** comprising the steps of:
- measuring local sun conditions;
- calculating an intensity value (I), representing local sun conditions, on the basis of the local sun condition measurement;
- measuring the light reflected by a user's skin surface;
- calculating a reflection value (R_{D}), representing the user's phototype and skin condition, on the basis of the measurement of the light reflected by the user's skin surface;
- calculating an optimum sun protection factor (SPF) as a function of said reflection value (R_{D}) and said intensity value (I).

9. A method as claimed in Claim 8, **characterized by** comprising the step of calculating a personalized sun exposure program as a function of said reflection value (R_{D}) and said intensity value (I); said program indicating one or more exposure periods, and, for each period, the time of day, the exposure time, and the sun protection factor to be used in that period.

10. A method as claimed in Claim 7 or 8, **characterized by** comprising the step of illuminating the user's skin surface with a light source, and measuring the intensity of the light emitted by the light source and reflected by the skin surface.

11. A method as claimed in one of Claims 8 to 10, **characterized by** comprising a step of daily adapting the sun protection factor to be used and the exposure duration and/or time interval to skin conditions and sun conditions.

12. A method as claimed in Claim 11, **characterized by** comprising the step of comparing the reflection value (R_{D}) of the current day with a reflection value (R_{D-1}) of the previous day, and for accordingly adapting the sun exposure program.
